# EUROPEAN PATENT APPLICATION

(11) **EP 3 733 845 A1**
(43) Date of publication of application: **04.11.2020**
(21) Application number: 18895451.5
(22) Date of filing: 28.12.2018
(51) Int. Cl.: C12N 15/10, C12Q 1/68

(54) **METHOD FOR RAPIDLY PREPARING SANGER SEQUENCING TEMPLATE**

(30) Priority: 28.12.2017 CN 201711460204
(71) Applicant: Nanjingjinsirui Science & Technology Biology Corp., Nanjing, Jiangsu 211100 (CN)
(72) Inventor: FAN, Long, Nanjing, Jiangsu 211100 (CN); ZHOU, Jingbo, Nanjing, Jiangsu 211100 (CN); JIANG, Haojun, Nanjing, Jiangsu 211100 (CN); LIU, Jiadong, Nanjing, Jiangsu 211100 (CN); WU, Cheng-Hsien, Nanjing, Jiangsu 211100 (CN)
(74) Representative: Pharma Patents International AG
(86) International application number: PCT/CN2018/124721
(87) International publication number: WO 2019/129185

(57) **Abstract**

A method for rapidly preparing a Sanger sequencing template, comprising: performing denaturing treatment on a sample containing circular DNAs; performing rolling circle amplification of the denaturing product; and treating the rolling circle amplification product with alkaline phosphatase to remove residual dNTP.

## Description

### Technical Field

The present invention relates to the field of biological engineering, and in particular, to a method for rapidly preparing a Sanger sequencing template.

### Background Art

Sanger sequencing method, i.e. dideoxy chain termination method, is a classic method for nucleic acid sequencing. Its basic principle is as follows: the introduction of dideoxynucleoside triphosphates (ddNTPs) into a reaction system, in which a complementary strand is synthesized by a DNA polymerase using a target DNA to be sequenced as a template, causes the complementary strand in the synthesis to terminate at target DNA nucleotides corresponding to ddNTPs, forming a series of strand termination fragments; polyacrylamide gel electrophoresis is used to distinguish various DNA strand termination fragments by only one nucleotide in length, and the type of the target DNA nucleotide at the corresponding position of the target DNA is determined according to the type of ddNTP at the end of each fragment

At present, conventional Sanger sequencing template preparation requires two steps of shaking culture and plasmid extraction (such as alkaline lysis or commercial mini extraction kit), which takes about 14 hours in total. There is a need in the art for improving the template sample preparation process of Sanger sequencing, and providing a sample preparation method that can shorten the preparation time while ensuring sequencing efficiency.

The rolling circle amplification (RCA) technology can be used to amplify circular DNA templates, such as plasmids and phage DNAs, for Sanger sequencing. This technology is a nucleic acid amplification technology established on the basis of the method of rolling circle replication of circular DNA molecules of pathogenic microorganisms in nature, and it is a DNA amplification technology that occurs at a constant temperature. In the RCA reaction, when there is a circular DNA template and a polymerase, replication occurs by the action of DNA polymerase with the circular DNA as a template, and finally a DNA single strand with repeated sequences complementary to the DNA template is formed (Lizardi *et al.,* 1998).

An RCA system comprises: (1) phage phi29 DNA polymerase; (2) a circular template (currently the RCA amplification template may also be linear); and (3) 1 or 2 primers, or a plurality of primers, which generally need to be resistant to endonuclease. Among these, the phage phi29 DNA polymerase is very important. It has high strand displacement activity, and can perform 70 kb strand displacement DNA synthesis without template separation. Moreover, it has a stable performance and a high continuous synthesis capacity, and can efficiently catalyze DNA synthesis for several hours at a synthesis speed of about 50 bp/s. In addition, the phi29 DNA polymerase has strong error correction ability, and the error rate is comparable to that of pfu enzyme, and significantly lower than that of Taq DNA polymerase. RCA is an isothermal signal amplification method with a linear amplification factor of 10⁵ and an exponential amplification capacity of greater than 10⁹. The RCA technology is a trace molecular detection method that can be used for the detection and research of extremely trace biological macromolecules and biomarkers. At present, this method has been successfully used for the amplification of linear double-stranded DNAs and even whole genomic DNAs (Esteban *et al.,* 1993; Qian *et al.,* 2003; Simison *et al.,* 2006). With its unique advantages, the RCA technology has shown great application prospects in the fields of gene detection, genetic diagnosis, in situ detection, immune detection, microarray solid-phase detection and the like.

### Summary of the Invention

One of the objectives of the present invention is to solve the problem that the existing Sanger sequencing template preparation cycle is relatively long.

In order to solve this problem, in one aspect, the present application provides a method for rapidly preparing a Sanger sequencing template, comprising: performing denaturing treatment on a sample containing circular DNAs; mixing a denatured product with a rolling circle amplification reaction solution to perform an isothermal rolling circle amplification; and treating a rolling circle amplification product with alkaline phosphatase to remove residual dNTPs.

In the context of the present application, "denaturing" is understood in accordance with the ordinary meaning of nucleic acid denaturation in the art According to some embodiments, the denaturing treatment comprises treating the samples at an elevated temperature until the denaturation is completed, and then lowering the temperature of the samples. According to some more specific embodiments, the denaturing treatment is performed by heating the samples to a temperature of about 95ºC for 3 minutes. According to some more specific embodiments, the samples are cooled to 4ºC after heating, and kept According to some further embodiments, random primers for the rolling circle amplification are added to a reaction system of the denaturing treatment. In some specific embodiments, the random primers are 7 bp in length. Without wishing to be bound by any theory, the high temperature can ensure the sufficient release of the initial template DNAs, and the cooling process can allow the random primer(s) and the circular DNA template to be annealed. By adding random primers before the denaturation process, compared with addition of random primers after the denaturation process, the effect of the rolling circle amplification can be further improved.

In some embodiments, the reaction system of the denaturing treatment comprises EDTA. In some preferred embodiments, the concentration of EDTA in the reaction system of the denaturing treatment is about 0.04 mM. In some specific embodiments, the denaturing treatment comprises the steps of: adding 1 ul of a sample containing circular DNAs to 2 ul of a denaturing buffer (10 mM Tris-HCl, 0.1 mM EDTA, pH 8.0, 25ºC), 2 ul of random primers (50-150 uM) to a total volume of 5 ul, mixing well, treating at 95ºC for 3 minutes to complete the denaturation, and cooling to 4ºC.

In some embodiments, the reaction system of the rolling circle amplification comprises phi29 DNA polymerase. In some further embodiments, the rolling circle amplification comprises an isothermal amplification at a temperature of 30ºC. In some further embodiments, the reaction system of the rolling circle amplification comprises bovine serum albumin (BSA). In some further embodiments, the concentration of bovine serum albumin (BSA) is about 0.1 mg/mL. In some embodiments, the reaction system of the rolling circle amplification comprises KCl. In some further embodiments, the concentration of KCl in the reaction system of the rolling circle amplification is about 75 mM.

According to some specific embodiments of the method of the present invention, the rolling circle amplification preferably comprises the steps of: to the product which has been subjected to the step of denaturing treatment, adding 1 ul of lOx amplification buffer (500 mM Tris-HCl, 25-75 mM MgCl₂, 500-1,000 mM KCl, 20-60 mM DTT, pH 8.2, at 25ºC), 0.1 ul of BSA (10 mg/ml), 1.6 ul of dNTPs (1-4 mM), 1 ul of phi29 DNA polymerase (5-15 U/ul), and 1.3 ul of ddH₂O, made up to 10 ul of the final total reaction system, mixing well, amplifying at 30ºC for 3 hours, treating at 65ºC for 10 minutes, and cooling to 4ºC.

In some embodiments, the alkaline phosphatase used for treating the rolling circle amplification product to remove residual dNTPs is shrimp alkaline phosphatase (rSAP). In some embodiments, the alkaline phosphatase treatment comprises treating a rolling circle amplification product in the presence of 0.01 to 0.1 U/uL, preferably 0.05 U/uL alkaline phosphatase, preferably shrimp alkaline phosphatase. In a specific embodiment, the treatment of the rolling circle amplification product comprises: to 10 ul of an amplification product, adding 2 ul of 10x CutSmart Buffer (250-750 mM potassium acetate, 100-300 mM Tris-acetic acid, 50-150 mM magnesium acetate, 1,000 µg/ml BSA, pH 7.9, 25ºC), 1 ul of shrimp alkaline phosphatase (0.5-1.5 U/ul), and 7 ul of ddH₂O, made up to 20 ul of the final total reaction system, reacting at 37ºC for 30 minutes, treating at 65ºC for 5 minutes, and cooling to 4ºC.

The treated amplification product can be directly used for Sanger sequencing, and in general, 1 ul can be used for PCR amplification, purification and on-line testing of conventional Sanger sequencing.

Therefore, in another aspect, the present invention provides a Sanger sequencing method, comprising: preparing a sample to be sequenced by the sample preparation method of the previous aspect of the present invention, and directly performing Sanger sequencing on a template sample prepared by the method. In a preferred embodiment, 1 ul of each prepared template sample is subjected to Sanger sequencing.

The sample applicable to the present invention may be any biological sample comprising or consisting substantially of circular DNAs, for example, without limitation, bacterial solutions, colonies, cell debris, plasmids, M13 phage, and any other circular DNA samples. The sample may or may not be purified before being applied to the method of the present invention.

### Beneficial Effects

The rolling circle amplification method used in the present invention is generated by optimizing based on the commercial reagents and the rolling circle amplification method reported in reference documents. This rolling circle amplification method optimizes the existing rolling ring amplification system, reagents and method flow as a whole; effectively solves the problem that the traditional sequencing template preparation cycle is too long, on the premise of ensuring that the sequencing effect is comparable to the sequencing effect in the case of preparing a template by a conventional extraction of plasmids or circular DNAs; and is thus an efficient method for rapidly preparing a sequencing template. The preparation of a conventional sequencing plasmid template takes about 14 hours from the initial shaking culture to the plasmid extraction and detection, and the conventional RCA method takes 16 hours. By optimizing the RCA, the present invention can complete the preparation of a high-quality sequencing template within 4 hours, greatly shortening the time for obtaining sequencing results, and greatly saving reagent and labor costs. In general, the rolling circle method for rapidly preparing a Sanger sequencing template provided by the present invention can effectively overcome shortcomings such as high cost, long consuming time and tedious steps of the traditional sequencing template preparation method, and its unique design, simple operation, and high sequencing success rate enable rapid and easy preparation of a Sanger sequencing template without relying on expensive instruments.

### Brief Description of the Drawings:

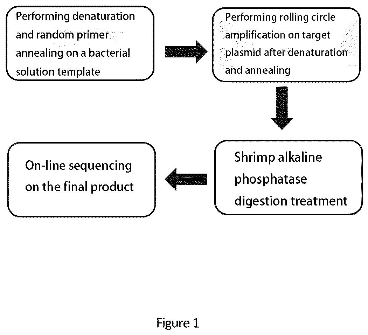

Figure 1 shows a flow chart of sequencing template preparation of the present invention.

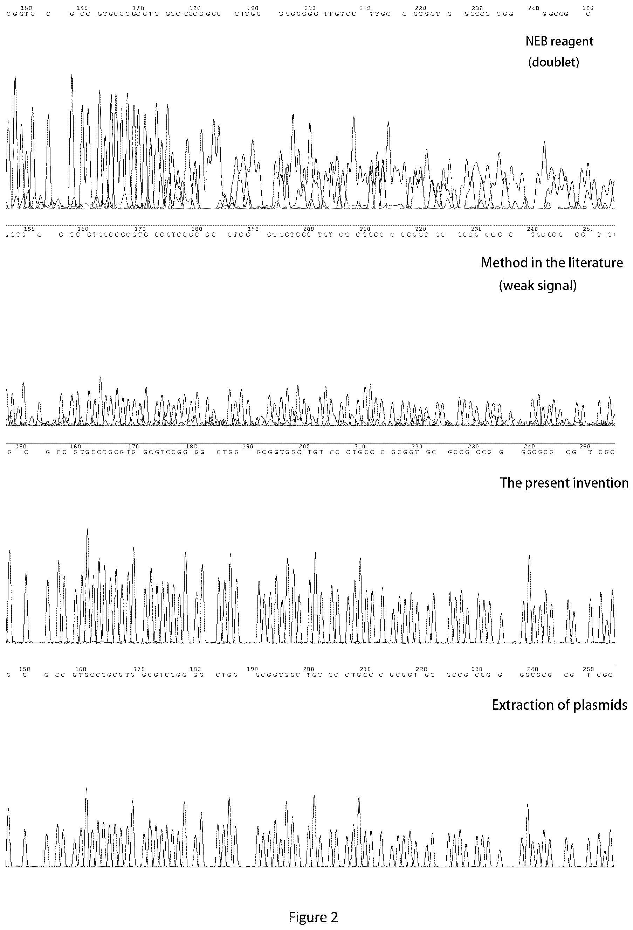

Figure 2 shows a comparison of typical sequencing results of pUC57 in Example 1.

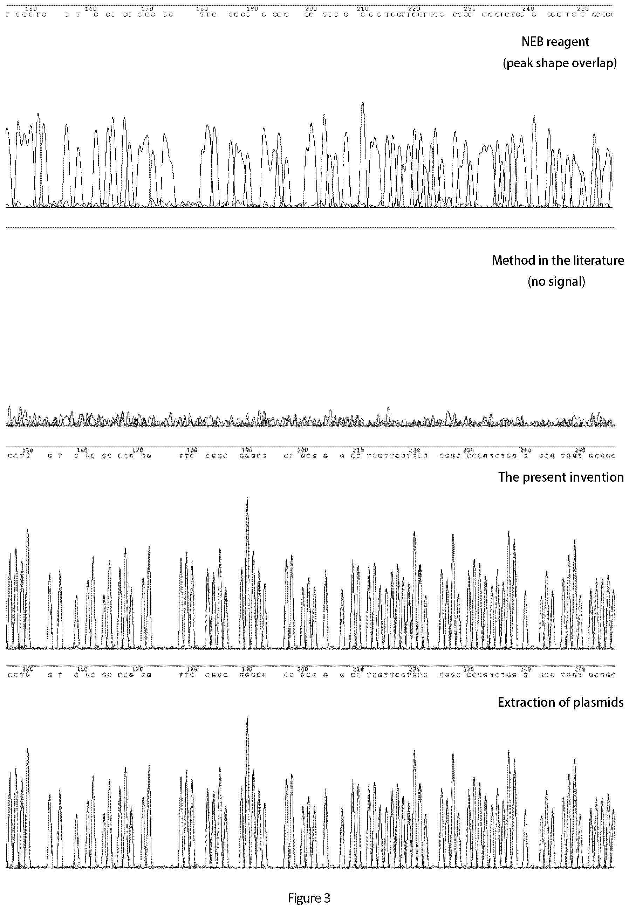

Figure 3 shows a comparison of typical sequencing results of pET in Example 1.

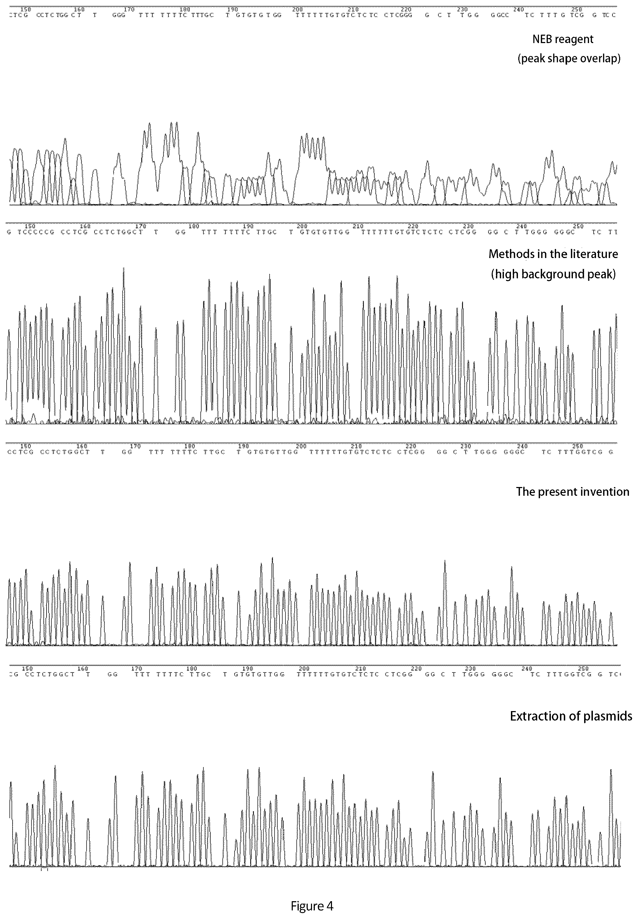

Figure 4 shows a comparison of typical sequencing results of pTGE5 in Example 1.

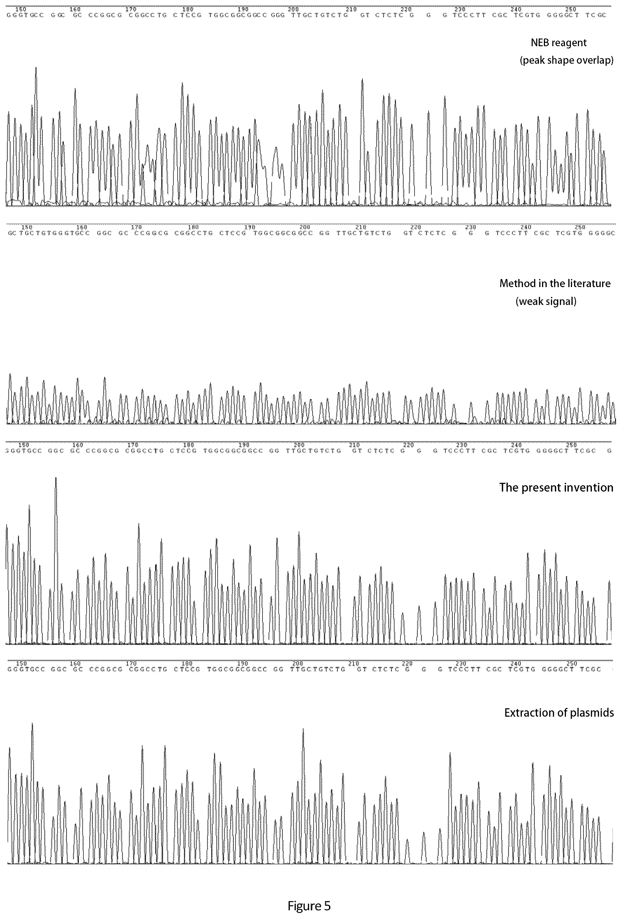

Figure 5 shows a comparison of typical sequencing results of pcDNA3.1 in Example 1.

### Detailed Description of Embodiments:

In order to further understand the method described in the present invention, the present invention will be further described below with reference to the accompanying drawings and examples.

### Example 1:

This example compares the sequencing effects of preparing a Sanger sequencing template within four hours by using three different rolling circle amplification methods for preparing a sequencing template:
A. NEB phi29 DNA polymerase reagent (Cat# M0269S, this reagent provides some supporting reagents and experimental protocols for RCA reactions) (hereinafter referred to as "NEB reagent (phi29 DNA polymerase)")
B. RCA experimental protocol cited from literature (Frank B. Dean., John R. Nelson., Theresa L. Giesler., and Roger S. Lasken. 2001. Rapid Amplification of Plasmid and Phage DNA Using Phi29 DNA Polymerase and Multiply-Primed Rolling Circle Amplification. Genome Research. 1095-1099) (hereinafter referred to as the "methods in the literature")
C. Optimized RCA solution provided by the present application (hereinafter referred to as "the present application")

The test samples were 32 bacterial solutions transformed with 4 different types of plasmids. The OD600 value of each bacterial solution was measured with a spectrophotometer, and the results are shown in Table 1.

Three different sequencing template preparation processes were used to prepare sequencing templates, as described below:

### 1) Step 1 (denaturation process): performing denaturation and random primer annealing on a bacterial solution template

The experimental samples were bacterial solutions transformed with plasmids as described above. The system configurations of denaturation reactions for three different sequencing template preparation methods are listed in the following table respectively:

| NEB reagent (phi29 DNA polymerase) | |
|---|---|
| Component | Volume (ul) |
| Bacterial solution | 1 |
| 10x reaction buffer (500 mM Tris-HCl, 100 mM MgCl₂, 100 mM (NH₄)₂SO₄, 40 mM DTT, pH 7.5, 25ºC] | 1 |
| 7 bp random primer (100 uM) | 2.5 |
| ddH₂O | 4.3 |
| Total volume | 8.8 |

| Methods in the literature | |
|---|---|
| Component | Volume (ul) |
| Bacterial solution | 2 |
| TE buffer (10 mM Tris-HCl, 1 mM EDTA, pH 8.0, 25ºC) | 8 |
| Total volume | 10 |

| Method of the present application | |
|---|---|
| Component | Volume (ul) |
| Bacterial solution | 1 |
| Denaturing buffer (10 mM Tris-HCl, 0.1 mM EDTA, pH 8.0, 25ºC) | 2 |
| 7 bp random primer (120 uM) | 2 |
| Total volume | 5 |

Three different sequencing template preparation methods used the same experimental operation flow of denaturation reaction, as shown in the following table:

| Temperature | Time (min) |
|---|---|
| 95ºC | 3 |
| 4ºC | Keeping |

### 2) Step 2 (amplification process): performing isothermal rolling circle amplification on target plasmid after denaturation and annealing

Using the reaction product from Step 1 as an experimental sample, the isothermal rolling circle amplification was performed according to three different sequencing template preparation methods. The system configuration of the amplification reaction in each method is shown in the following table:
The system configurations of amplification reactions in three different sequencing template preparation methods:

| **NEB reagent (phi29 DNA polymerase)** | |
|---|---|
| Component | Volume (ul) |
| Reaction product from Step 1 | 8.8 |
| dNTP (10 mM) | 0.5 |
| BSA (10 mg/mL) | 0.2 |
| Phi29 DNA polymerase (10 U/uL) | 0.5 |
| Total volume | 10 |

| **Methods in the literature** | |
|---|---|
| Component | Volume (ul) |
| Reaction product from Step 1 | 10 |
| 10x amplification buffer (500 mM Tris-HCl, 50 mM MgCl₂, 750 mM KCl, 1 mM DTT, pH 8.2, 25ºC) | 2 |
| 7 bp random primer (100 uM) | 1 |
| dNTP (2.5 mM) | 0.8 |
| Phi29 DNA polymerase (10 U/ul) | 0.5 |
| Yeast pyrophosphatase (0.1 U/ul) | 0.3 |
| ddH₂O | 5.4 |
| Total volume | 20 |

| **Method of the present application** | |
|---|---|
| Component | Volume (ul) |
| Reaction product from Step 1 | 5 |
| 10x reaction buffer (500 mM Tris-HCl, 50 mM MgCl₂, 750 mM KCl, 50 mM DTT, pH 8.2, 25ºC) | 1 |
| BSA (10 mg/mL) | 0.1 |
| dNTP (2.5 mM) | 1.6 |
| Phi29 (10 U/uL) | 1 |
| ddH₂O | 1.3 |
| Total volume | 10 |

Three different sequencing template preparation methods used the same experimental operation flow of isothermal amplification, as shown in the following table:

| Temperature | Time |
|---|---|
| 30ºC | 3 hours |
| 65ºC | 10 minutes |
| 4ºC | Keeping |

### 3) Step 3 (digestion process)

The method provided by the present application further comprises a shrimp alkaline phosphatase digestion treatment step after the isothermal amplification. The experimental sample in this step was the amplification product from Step 2.

The system configuration of the shrimp alkaline phosphatase treatment reaction is as follows:

| Component | Volume (ul) |
|---|---|
| Amplification product from Step 2 | 10 |
| 10x Cut Smart Buffer (520 mM potassium acetate, 200 mM Tris-acetic acid, 120 mM magnesium acetate, 1,000 µg/ml BSA, pH 7.9, 25ºC) | 2 |
| Shrimp alkaline phosphatase (rSAP) (1 U/uL) | 1 |
| ddH₂O | 7 |
| Total volume | 20 |

The experimental operation flow of the shrimp alkaline phosphatase treatment reaction is as follows:

| Temperature | Time (min) |
|---|---|
| 37ºC | 30 |
| 65ºC | 5 |
| 4ºC | Keeping |

### 4) On-line sequencing

1 ul of an equal-concentration sequencing template prepared by each of the three different preparation methods described above within 4 hours was taken for Sanger sequencing, and compared with the sequencing result from standard shaking culture and extraction of plasmids (which took 14 hours). We found that for different plasmid types, the sequencing results of the template prepared by NEB reagent have the phenomena of doublet signals (i.e. fluorescence signals being superposed) and peak shape overlap (i.e. the peak shapes being not separate); the method reported in the literature has the phenomena of no signal, weak sequencing signals (i.e. the peak height being too low) and high background peak (i.e., the background signal value being too high); and only the RCA method optimized in the present application is consistent with the sequencing effect obtained by standard shaking culture plus extraction of plasmids (i.e. the main signal peak being clear and independent, and the background peak value being low).

This example utilizes the optimized method provided by the present application to successfully complete the preparation of a sequencing template within 4 hours, with a sequencing success rate of 100%. Meanwhile, we also compared the differences between the NEB reagent product method, the method reported in the literature and the method of the present invention in the template prepared within the same 4 hours. The sequencing success rate of the method of the present invention is much higher as compared with the other two methods (see Table 1), and is consistent with the sequencing effect obtained by standard shaking culture and extraction of plasmids (which takes 14 hours). It generally takes 16 hours to achieve the same effect in the case of using an unoptimized RCA to prepare a Sanger sequencing template. It indicates that the present method has great advantages in the rapid preparation of plasmid sequencing templates (that is, the sequencing template preparation time is shortened to within 4 hours on the premise that the sequencing success rate remains unchanged).

**Table 1: Comparison of sequencing success rates in Example 1**

| Sample type | Sample number | Sequencing success rate | | |
|---|---|---|---|---|
| | | NEB reagent | Method in the literature | Method of the present application |
| pUC57 series bacterial solution | 8 | 62.5% | 0% | 100% |
| pET series bacterial solution | 8 | 12.5% | 0% | 100% |
| pTGE5 series bacterial solution | 8 | 75% | 75% | 100% |
| pcDNA3.1 series bacterial solution | 8 | 100% | 50% | 100% |

## Claims

1. A method for rapidly preparing a Sanger sequencing template, comprising the steps of: performing denaturing treatment on a sample containing circular DNAs; performing rolling circle amplification of the denaturing product; and treating a rolling circle amplification product with alkaline phosphatase to remove residual dNTPs.

2. The method of claim 1, wherein the samples are mixed with random primers for the rolling circle replication before starting the denaturing treatment

3. The method of claim 1 or 2, wherein the denaturing treatment comprises heating the samples to 95ºC and keeping for 3 minutes, and then cooling to 4ºC and keeping.

4. The method of any one of claims 1 to 3, wherein a system of the denaturing treatment comprises EDTA, preferably about 0.04 mM EDTA.

5. The method of any one of claims 1 to 4, wherein a reaction system of the rolling circle amplification comprises bovine serum albumin, preferably about 0.1 mg/mL bovine serum albumin.

6. The method of any one of claims 1 to 5, wherein the reaction system of the rolling circle amplification comprises KCl, preferably about 75 mM KCl.

7. The method of any one of claims 1 to 6, wherein the rolling circle amplification comprises an isothermal amplification at a temperature of 30ºC for 3 hours.

8. The method of any one of claims 1 to 7, wherein the alkaline phosphatase treatment comprises treating a rolling circle amplification product at 37ºC, in the presence of 0.01 to 0.1 U/uL, preferably 0.05 U/uL shrimp alkaline phosphatase, preferably for 30 min.

9. The method of any one of claims 1 to 8, wherein the entire preparation process according to the method is completed within 4 hours.

10. A Sanger sequencing method, comprising: preparing a sample to be sequenced by the method of any one of claims 1 to 9, and directly performing Sanger sequencing on a template sample prepared by the method.
